# EUROPEAN PATENT SPECIFICATION

(11) **EP 1 411 971 B1**
(45) Date of publication and mention of the grant of the patent: **29.03.2006**
(21) Application number: 02758773.2
(22) Date of filing: 18.07.2002
(51) Int. Cl.: A61K 38/17, A61K 48/00, A61K 49/00, A61P 15/08

(54) **USE OF LONG PENTRAXIN PTX3 FOR TREATING FEMALE INFERTILITY**
VERWENDUNG VON LONG PENTRAXIN PTX3 FÜR DIE BEHANDLUNG VON INFERTILITÄT BEI FRAUEN
UTILISATION DE LONGUE PENTRAXINE PTX3 AUX FINS DE TRAITEMENT DE L'INFERTILITE FEMININE

(30) Priority: 03.08.2001 US 309472 P
(43) Date of publication of application: 28.04.2004
(73) Proprietor: SIGMA-TAU Industrie Farmaceutiche Riunite S.p.A., 00144 Roma (IT)
(72) Inventor: MANTOVANI, A., Istituto di Ricerche Farmacologiche, 20157 Milan (IT)
(74) Representative: Spadaro, Marco
(86) International application number: PCT/IT2002/000473
(87) International publication number: WO 2003/011326

(56) References cited:
- WO-A-99/32516
- VARANI SIMONA ET AL: "Knockout of pentraxin 3, a downstream target of growth differentiation factor-9, causes female subfertility." MOLECULAR ENDOCRINOLOGY, vol. 16, no. 6, June 2002 (2002-06), pages 1154-1167, XP009002022 June, 2002 ISSN: 0888-8809

## Description

### Background of the Invention

This invention relates to the requirement of PTX3 activity for female fertility. A genetic mutation which reduces PTX3 activity results in female sterility.

Pentraxins are a superfamily of proteins, which is characterized by a cyclic multimeric structure [1]. The classical short pentraxins C-reactive protein (CRP) and serum amyloid P component (SAP) are acute phase proteins in man and mouse, respectively, produced in the liver in response to inflammatory mediators; in particular, they are directly induced by interleukin-6 [2-3].

Long pentraxins share similarities with the classical short pentraxins, but differ by the presence of an unrelated long N-terminal domain coupled to the C-terminal pentraxin domain, as well by genomic organization, chromosomal localization, cellular source, inducing stimuli, and ligands recognized. Long pentraxin 3 (PTX3) is the first long pentraxin identified as an interleukin-1 (IL-1) inducible gene in endothelial cells [4] and as a tumor necrosis factor-α (TNFα)) inducible gene in fibroblasts [5]. PTX3 is also produced by macrophages and other cell types and tissues upon stimulation with primary inflammatory mediators (LPS, IL-1, TNFα) [6-8]. PTX3 consists of a C-terminal 203-amino acid pentraxin-like domain and an N-terminal 178-amino acid unrelated domain. When secreted, glycosylated PTX3 protomers (45 kDa) assemble to form 10-20 multimers [9]. PTX3 does not bind to classical pentraxin ligands such as phosphoethanolamine, phosphocholine, high pyruvate agarose, collagen IV, fibronectin, or gelatin. In contrast, PTX3 specifically binds with high affinity to C1q by the pentraxin domain [9]. PTX3 plasma levels are very low in normal conditions (≤ 2 ng/ml) but increase in several pathological conditions (10-100 ng/ml) including infections [10].

Other long pentraxins cloned after PTX3 include guinea pig apexin [11, 12] which is expressed in the sperm acrosome, XL-PXN1 from Xenopus laevis [13], rat neuronal pentraxin 1 (NP1) [14], human NP1 and NP2 [15, 16], mouse NP1 and NP2 [15], Narp [17], and neuronal pentraxin receptor (NRP), a putative integral membrane pentraxin [18-9]. The in vivo function of long pentraxins has not been unequivocally defined.

PTX3 consists of two structural domains: a N-terminal domain unrelated to any known molecule and a C-terminal domain similar to the short pentraxins such as C-reactive protein (Breviario et al., J. Biol. Chem., 267:22190-22197, 1992). Substantial similarity has been found between human PTX3 (hPTX3) and mouse PTX3 (mPTX3). The degree of identity between human and murine PTX3 genes is 82%, and reaches 90% if conservative substitutions are considered (Introna et al., Blood, 87:1862-1872, 1996). The genes are located in syntenic chromosome locations. The high degree of similarity between hPTX3 and mPTX3 sequences is a sign of the high degree of conservation of pentraxins during evolution (Pepys & Baltz, Adv. lmmunol., 34:141-212, 1983). Pentraxins are reviewed by Gewurz et al. (Curr. Opin. lmmunol., 7:54-64, 1995).

WO 99/32516 describes the use of PTX3 for the therapeutic treatment of cancer, inflammation, and infectious diseases.

US Patent 5,767,252 describes a growth factor for neuronal cells belonging to the pentraxin family.

WO 02/36151 describes the use of PTX3 for the preparation of medicament for the prevention and treatment of autoimmune pathologies.

Varani, S., et al.; Mol. Endocrinol., 2002, Jun; 16(6):1154-67 report that females knockout of pentraxin 3 are subfertile, showing an important role of PTX3 in cumulus-oocyte interaction in the preovulatory period as a downstream protein in the GDF-9 signal transduction cascade.

In contrast to the foregoing, the study of mice genetically modified at their PTX3 genetic locus, which were produced by homologous recombination in embryonic stem cells, and the effects thereof has revealed the involvement of PTX3 activity in female fertility.

It is an objective of the invention to use the recombinant human PTX3 for a medicament for increasing female fertility. The effects of female sterility may be ameliorated, reproductive ability may be increased or decreased as desired, female fertility may be enhanced, or combinations thereof. Other treatments such as in vitro fertilization require invasive procedures and complicated technology. The need for therapies that affect female fertility is thereby addressed. Other advantages and improvements are discussed below, or would be apparent from the disclosure herein.

Pharmaceutical compositions, methods for using and making them, and further objectives are described below.

### Summary of the Invention

An object of the invention is to provide a medicament which is comprised of recombinant human PTX3 in an amount sufficient to increase reproductive ability in a female subject with a defect in reproduction. The discovery that PTX3 activity is required may be used as therapy of a female patient or animal with a defect in reproduction or for diagnosis of her ability to reproduce.

The subject may be a female patient or animal. The composition may be suitable for systemic administration or adapted for local administration (i.e., within or around a female reproductive organ). The composition may be used to treat sterility.

Another object of the invention is to provide methods of administering the pharmaceutical composition to a subject in need of treatment for female sterility in an amount sufficient to increase the subject's reproductive ability.

Detecting PTX3 in a female subject and correlating this amount with her reproductive ability is a further objective of the invention. Mutations in the human PTX3 genetic locus would map to chromosome 3q24-q28; mutations in interacting genes would map outside the PTX3 genetic locus. The function of a PTX3 variant may be determined by comparison to known PTX3 sequences or other pentraxin sequences; folding, glycosylation, secretion, or formation of multimers; receptor binding or signal transduction; effect on reproductive ability, fertility, or sterility; or combinations thereof.

Further aspects of the invention will be apparent to a person skilled in the art from the following description and claims, and generalizations thereto.

### Brief Descriptions of the Drawings and Sequence Listing

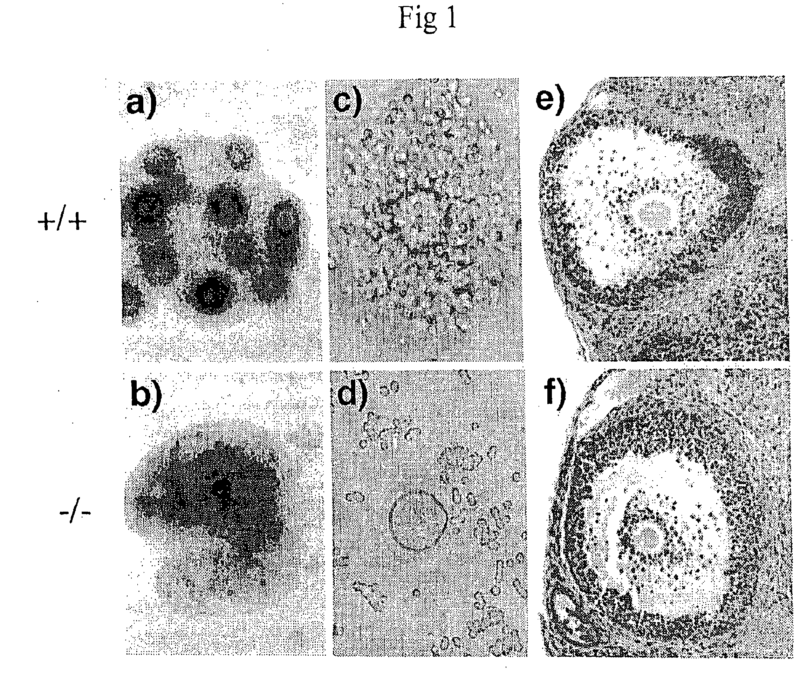

Figs. 1A-1F illustrate the abnormal morphology of cumuli oophori from PTX3 -/- mice. Cumuli oophori were recovered 14-16 hr after hCG treatment. They are shown after collection (A and B) or 4 hr later (C and D). In PTX3 +/+ mice (A and C), granulosa cells form a compact and stable cumulus around the oocyte (arrow da mettere). In PTX3 -/- mice (B and D), they are loosely associated to the oocyte and the cumulus has completely disappeared in 4 hr. Histological examination of the ovaries of PTX3 +/+ (E) and PTX3 -/- (F) mice shows normal antral follicles.

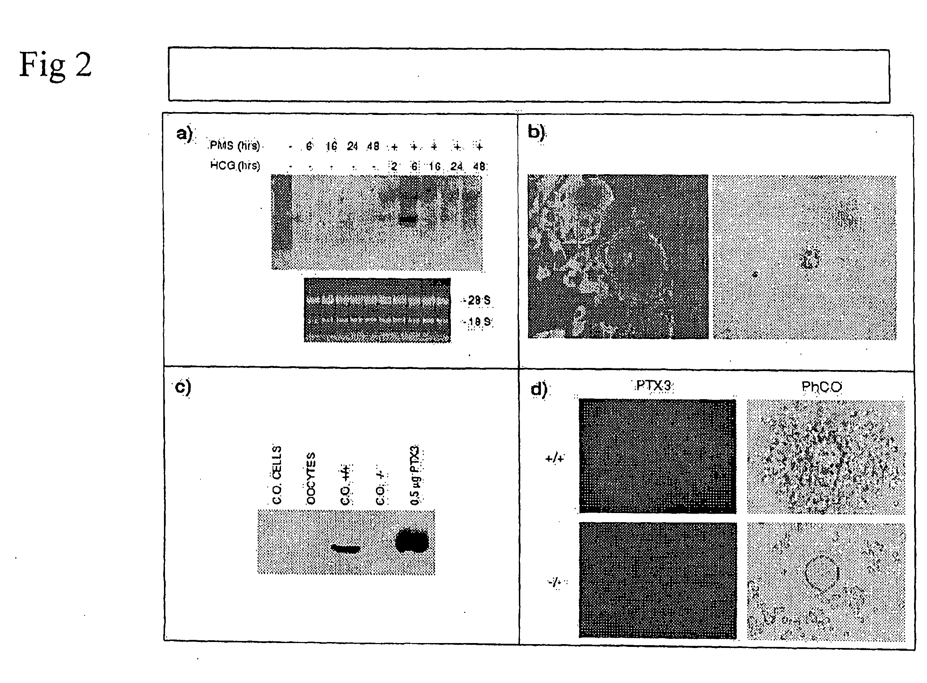

Figs. 2A-2D show PTX3 mRNA and protein expression in ovarian tissue. (A) Kinetics of PTX3 expression in ovary after hormonally-induced superovulation (PMS treatment followed 48 hr later by hCG treatment) were shown at the mRNA level. Ovaries were collected at 0, 6, 16, 24 or 48 hr after PMS treatment and then 2, 6, 16, 24 or 48 hr after hCG treatment. Ten µg of total RNA was loaded in each lane. Ethidium bromide staining of the gel is shown in the lower panel. (B) In situ hybridization of the ovary: granulosa cell express PTX3 mRNA only in mature follicles. (C) PTX3 expression by cumuli oophori (C.O.), cumulus oophorus cells (C.O. cells), and oocytes was detected by Western blotting. Cumuli oophori were recovered from four PTX3 +/+ and PTX3 -/- superovulated females; cumulus oophorus cells and oocytes were obtained from seven and 14 PTX3 +/+ superovulated females, respectively. (D) Phase contrast (right panels) and immunofluorescence analysis (left panels) of cumuli oophori from PTX3 -/- (lower panels) and PTX3 +/+ (upper panels) mice are illustrated.

Sequences of a human cDNA and its translated open reading frame (SEQ ID NOS:1-2, respectively), a mouse cDNA and its translated open reading frame (SEQ ID NOS:3-4, respectively), human and mouse upstream regulatory regions (SEQ ID NOS:5-6, respectively), and PCR primers (SEQ ID NOS:7-10) are shown in the Sequence Listing. Alignment of human and mouse amino acid sequences shows 312 of 381 residues are identical (82%) and 351 residues are at least similar (92%). Both genes contain three exons: the first encodes for 43 amino acid residues, the second encodes for 135 amino acid residues with no high similarity to known sequence motifs, and the third encodes 203 amino acid residues with similarity to pentraxins. A pentraxin-like domain includes two Cys residues at positions 162 and 254 and a consensus "pentraxin-like" sequence His-Xaa-Cys-Xaa-Ser/Thr-Trp-Xaa-Ser (SEQ ID NO:11).

### Description of Specific Embodiments of the Invention

Native PTX3 is glycosylated (potential N-linked glycosylation site at position 203). A multimeric PTX3 complex eluted in gel filtration with a relative molecular weight of about 900 kDa. It migrated in gel electrophoresis under nondenaturing and nonreducing conditions as a predominant band of about 440 kDa (e.g., 9- or 10-mer of about 45 kDa protomers) with two minor bands in the 540-600 kDa range. Circular dichroism analysis indicated that PTX3 contained mostly β-sheet structure with some α-helical structure. PTX3 polypeptide or a complex thereof may be identified, isolated, or detected indirectly though a binding molecule (*e.g*., antibody, natural or nonnatural peptide mimetic) for the PTX3 gene product.

PTX3 polypeptide and its variants (i.e., deletion, domain shuffling or duplication, insertion, substitution, or combinations thereof) are also useful for determining structure-function relationships (e.g., alanine scanning, conservative or nonconservative amino acid substitution). For example, folding and processing of PTX3 protein, secretion of PTX3 protomer and formation of multimers, ligand binding to receptor, signal transduction, or combinations thereof. See Wells (Bio/Technology, 13:647-651, 1995) and U.S. Patent 5,534,617. Directed evolution by random mutagenesis or gene shuffling using PTX3 may be used to acquire new and improved functions in accordance with selection criteria. Mutant, polymorphic, and analog PTX3 polypeptides are encoded by suitable mutant, polymorphic, and analog PTX3 polynucleotides. Structure-activity relationships of PTX3 may be studied (i.e., SAR studies) using variant polypeptides produced by an expression construct transfected in a host cell with or without endogenous PTX3. Thus, mutations in discrete domains of the PTX3 polypeptide may be associated with decreasing or even increasing activity in the protein's function.

A PTX3 nucleotide sequence can be used to produce a fusion polypeptide with at least one heterologous peptide domain (e.g., an affinity or epitope tag). Oligopeptide is useful for producing specific antibody and epitope mapping of PTX3-specific antibody. A polypeptide may be at least 10, 15, 20, 25, 30, 35, 40, 45, 50, 60, 70, 80, 90, 100, 150, or more amino acids long (including intermediate ranges thereof). Oligopeptide may be conjugated to one affinity tag of a specific binding pair (e.g., antibody-digoxygenin/hapten/peptide, biotin-avidin/streptavidin, glutathione S transferase-glutathione, maltose binding protein-maltose, protein A or G/immunoglobulin, polyhistidine-nickel). Either a full-length PTX3 polypeptide (e.g., SEQ ID NO:2 or 4) or a shorter fragment (e.g., N-terminal or C-terminal domain) can be produced; optionally including a heterologous peptide domain. PTX3 polypeptide may be synthesized by chemical means, purified from natural sources, synthesized in transfected host cells, or combinations thereof.

The PTX3 nucleotide sequence or a portion thereof can be used to monitor PTX3 expression, to determine PTX3 sequence, and/or to detect PTX3 variants. The invention also provides hybridization probes and amplification primers (e.g., polymerase chain reaction, ligation chain reaction, other isothermal amplification reactions). A pair of such primers may be used for RT-PCR assays to quantitate PTX3 transcript abundance within cells. Amplification primers may be between 15 and 30 nucleotides long (preferably about 25 nucleotides), anneal to either sense or antisense strand (preferably the pair will be complementary to each strand), and terminate at the 3' end anywhere within SEQ ID NOS:1, 3 and 5-6 or their complements. Therefore, this invention will be useful for development and utilization of PTX3 primers and other oligonucleotides to quantitate cognate RNA and DNA within cells.

Binding of polynucleotides or polypeptides may take place in solution or on a substrate. The assay format may or may not require separation of bound from not bound. Detectable signals may be direct or indirect, attached to any part of a bound complex, measured competitively, amplified, or combinations thereof. A blocking or washing step may be interposed to improve sensitivity and/or specificity. Attachment of a polynucleotide or polypeptide, interacting protein, or binding molecule to a substrate before, after, or during binding results in capture of an unattached species. Such immobilization will be stably attached to the substrate under washing conditions. See US Patents 5,143,854 and 5,412,087.

Changes in gene expression may be manifested in the cell by affecting transcriptional initiation, transcript stability, translation of transcript into protein product, protein stability, glycoprotein processing, rate of folding or secretion, or combinations thereof. The gene, transcript, or polypeptide can also be assayed by techniques such as in vitro transcription, in vitro translation, Northern hybridization, nucleic acid hybridization, reverse transcription-polymerase chain reaction (RT-PCR), run-on transcription, Southern hybridization, metabolic protein labeling, antibody binding, immunoprecipitation (IP), enzyme linked immunosorbent assay (ELISA), radioimmunoassay (RIA), fluorescent labeling or histochemical staining, microscopy and digital image analysis, and fluorescence activated cell analysis or sorting.

A reporter or selectable marker gene whose product is easily assayed may be used for convenient detection. Reporter genes include, for example, alkaline phosphatase, β-galactosidase (LacZ), chloramphenicol acetyltransferase (CAT), β-glucoronidase (GUS), luciferases (LUC), green and red fluorescent proteins (GFP and RFP, respectively), horseradish peroxidase (HRP), β-lactamase, and derivatives thereof (e.g., blue EBFP, cyan ECFP, yellow-green EYFP, destabilized GFP variants, stabilized GFP variants, or fusion variants sold as LIVING COLORS fluorescent proteins by Clontech). Reporter genes would use cognate substrates that are preferably assayed by a chromogen, fluorescent, or luminescent signal. Alternatively, assay product may be tagged with a heterologous epitope (e.g., FLAG, MYC, SV40 T antigen, glutathione transferase, polyhistidine, maltose binding protein) for which cognate antibodies or affinity resins are available. Examples of drugs for which selectable marker genes, which confer resistance, exist are ampicillin, geneticin/kanamycin/neomycin, hygromycin, puromycin, and tetracycline. A metabolic enzyme (e.g., dihydrofolate reductase, HSV-1 thymidine kinase) may be used as a selectable marker in sensitive host cells or auxotrophs. For example, methotrexate can increase the copy number of a polynucleotide linked to a DHFR selectable marker or gancyclovir can negatively select for a viral thymidine kinase selectable marker.

A polynucleotide may be ligated to a linker oligonucleotide or conjugated to one member of a specific binding pair (e.g., antibody-digoxygenin/hapten/peptide epitope, biotin-avidin/streptavidin, glutathione S transferase or GST-glutathione, lectin-sugar, maltose binding protein-maltose, polyhistidine-nickel, protein A/G-immunoglobulin). The polynucleotide may be conjugated by ligation of a nucleotide sequence encoding the binding member. A polypeptide may be joined to one member of the specific binding pair by producing the fusion encoded by such a ligated or conjugated polynucleotide or, alternatively, by direct chemical linkage to a reactive moiety on the binding member by chemical cross-linking. Such polynucleotides and polypeptides may be used as an affinity reagent to identify, to isolate, and to detect interactions that involve specific binding of a transcript or protein product of the expression vector. Before or after affinity binding of the transcript or protein product, the member attached to the polynucleotide or polypeptide may be bound to its cognate binding member. This can produce a complex in solution or immobilized to a support. A protease recognition site (e.g., for enterokinase, Factor Xa, ICE, secretases, thrombin) may be included between adjoining domains to permit site specific proteolysis that separates those domains and/or inactivates protein activity.

Probes and primers may be used to identify a PTX3 gene or variant thereof. For example, a probe or primer specific for a human PTX3 gene identified herein may be used to detect the presence or absence of the gene, and thereby infer that the source of the gene is present or absent, respectively. Genetic polymorphisms and mutations in the PTX3 gene may be specifically detected by positioning a potentially mismatched base(s) in the middle portion of a probe or the 3'-end of a primer to stabilize or to destabilize binding of the probe or primer to its target depending on whether the target's sequence at that position is complementary to the base or not, respectively.

Genetic polymorphisms and mutations may also be detected by a change in the length of a restriction fragment (RFLP), nuclease-protected fragment (e.g., S1 nuclease, deoxyribonuclease I, ribonuclease A, H or T1), or amplified product. For complicated genetic fingerprints, identification of each component may not be needed because a side-by-side visual comparison might easily detect differences (e.g., RAPD). Differences may also be detected by changes in the molecular weight (MW) or isoelectric point (pl) of the PTX3 protein by gel electrophoresis or isoelectric focusing, respectively.

Presence of PTX3 protein may be used as an indication of PTX3 activity in human or animal fluids or tissues. The fluid may be blood, blood product (e.g., plasma, serum), lavage, sputum, or the like. Exemplary tissues are those of the epithelium (e.g., lung) or mucosa (e.g., mouth, vagina), although infection may be systemic and involve other tissue types as well. Signal may be detected in situ for solid tissue, on dispersed or homogenized tissue, in solution (e.g., diluted or undiluted body fluid, wash), or on a cell smear or touch prep. Oocyes which may be fertilized can be selected by PTX3 expression.

### Construction of Shuttle or Expression Vectors

A shuttle or expression vector is a recombinant polynucleotide that is in chemical form either deoxyribonucleic acid (DNA) and/or ribonucleic acid (RNA). The physical form of the vector may be single-stranded or double-stranded; its topology may be linear or circular. The vector is preferably a double-stranded deoxyribonucleic acid (dsDNA) or is converted into a dsDNA after introduction into a cell (e.g., insertion of a retrovirus into a host genome as a provirus). The vector may include one or more regions from a mammalian, insect, plant or fungal gene or a virus (e.g., adenovirus, adeno-associated virus, cytomegalovirus, fowlpox virus, herpes simplex virus, lentivirus, Moloney leukemia virus, mouse mammary tumor virus, Rous sarcoma virus, SV40 virus, vaccinia virus), as well as regions suitable for genetic manipulation (e.g., selectable marker, linker with multiple recognition sites for restriction endonucleases, promoter for in vitro transcription, primer annealing sites for in vitro replication). The vector may be associated with proteins and other nucleic acids in a carrier (e.g., packaged in a viral particle) or condensed with a chemical (e.g., cationic polymer) to target entry into a cell or tissue. Choice of vector polynucleotides and methods for introducing them into the female reproductive system (e.g., endometrium, ovary) is within the skill in the art.

An expression vector may be further comprised of a regulatory region for gene expression (e.g., promoter, enhancer, silencer, splice donor or acceptor site, polyadenylation signal, cellular localization sequence). Different levels of transcription can be achieved using an agent with a regulatory system which responds

Antibody specific for PTX3 can be used for inhibition or detection. Polyclonal or monoclonal antibodies may be prepared by immunizing animals (e.g., chicken, hamster, mouse, rat, rabbit, goat, horse) with antigen, and optionally affinity purified against the same or a related antigen. Antigen may be native protein, fragment made by proteolysis or genetic engineering, fusion protein, or in vitro translated or synthesized protein which includes at least one or more epitopes bound by the antibody. Antibody fragments may be prepared by proteolytic cleavage or genetic engineering; humanized antibody and single-chain antibody may be prepared by transplanting sequences from antigen binding domains of an antibody to framework molecules. Other binding molecules (e.g., agonists or antagonists of ligand-receptor binding) may be prepared by screening a combinatorial library for a member which specifically binds antigen (e.g., phage display library). Antigen may be a full-length protein encoded by the gene or fragment(s) thereof. The antibody may be specific for PTX3 or it may cross react with other pentraxins depending on how well the epitope recognized by the antibody is conserved among different species. See, for example, US Patents 5,403,484; 5,723,286; 5,733,743; 5,747,334; and 5,871,974.

PTX3-specific binding agents (e.g., polynucleotides, polypeptides) may be used diagnostically to detect PTX3 nucleic acid or protein, or for treatment to inhibit PTX3 activity (e.g., transcription, translation, processing, secretion, receptor binding). In particular, agents that affect PTX3 transcription and PTX3 binding to a receptor are desirable.

Compounds of the invention or derivatives thereof may be used as a medicament or used to formulate a pharmaceutical composition with one or more of the utilities disclosed herein.

Is therefore an object of the present invention the use of the recombinant human PTX3 for preparing a medicament for increasing the reproductive ability in a female subject with a defect in reproduction.

A further object of the present invention is a method for the determination of the reproductive ability in human female comprising the detection of the presence of PTX3 protein as diagnostic maker. affect the reproductive ability in a female subject

The compounds of the present invention may be administered in vitro to cells in culture, in vivo to cells in the body, or ex vivo to cells outside of a subject which may then be retumed to the body of the same subject or another. The subject is a female of reproductive age; she wants to become pregnant.

Compounds or derivatives thereof may be used to produce a medicament or other pharmaceutical compositions. Use of compositions which further comprise a pharmaceutically acceptable carrier and compositions which further comprise components useful for delivering the composition to a subject are known in the art. Addition of such carriers and other components to the composition of the invention is well within the level of skill in this art

A pharmaceutical composition may be administered as a formulation which is adapted for direct application to the female reproductive system (e.g., endometrium, ovary) or suitable for passage through the gut or blood circulation. Alternatively, pharmaceutical compositions may be added to the culture medium. In addition to active compound, such compositions may contain pharmaceutically-acceptable carriers and other ingredients known to facilitate administration and/or enhance uptake. The composition may be administered in a single dose or in multiple doses which are administered at different times.

Pharmaceutical compositions may be administered by any known route. By way of example, the composition may be administered by a mucosal, pulmonary, topical, or other localized or systemic route (e.g., enteral and parenteral). In particular, achieving an effective amount of PTX3 activity in or around the reproductive system may be desired. This may involve use of local application, implantation near a reproductive organ, or vaginal suppository. The term "parenteral" includes subcutaneous, intradermal, intramuscular, intravenous, intraarterial, intrathecal, and other injection or infusion techniques, without limitation.

Suitable choices in amounts and timing of doses, formulation, and routes of administration can be made with the goals of achieving a favorable response in the subject (i.e., efficacy), and avoiding undue toxicity or other harm thereto (i.e., safety). Therefore, "effective" refers to such choices that involve routine manipulation of conditions to achieve a desired effect: e.g., affecting reproductive ability, enhancing fertility, or reducing fertility.

A bolus of the formulation administered to a female subject once a day is a convenient dosing schedule. Alternatively, an effective dose may be administered every other day, once a week, or once a month. Dosage levels of active ingredients in a pharmaceutical composition can also be varied so as to achieve a transient or sustained concentration of the compound or derivative thereof in a subject and to result in the desired therapeutic response. But it is also within the skill of the art to start doses at levels lower than required to achieve the desired therapeutic effect and to gradually increase the dosage until the desired effect is achieved.

Dosing may be timed relative to the female subject's reproductive cycle (e.g., menses). As a practical matter, body temperature or hormone levels may be used as surrogates for events like ovulation and menstruation in reproduction.

The amount of compound administered is dependent upon factors such as, for example, bioactivity and bioavailability of the compound (e.g., half-life in the body, stability, and metabolism); chemical properties of the compound (e.g., molecular weight, hydrophobicity, and solubility); route and scheduling of administration; and the like. It will also be understood that the specific dose level to be achieved for any particular subject may depend on a variety of factors, including age, health, medical history, weight, combination with one or more other drugs, and severity of disease.

The term "treatment" refers to, inter alia, reducing or alleviating one or more symptoms of sterility in an affected subject. For a given subject, improvement in a symptom, its worsening, regression, or progression may be determined by an objective or subjective measure. Treatment may also involve combination with other existing modes of treatment and agents (e.g., superovulation). Thus, combination treatment may be practiced.

### EXAMPLES

Heterozygous females and males mice genetically modified for the PTX3 gene are normal and fertile. Breeding inter se yielded the predicted number of homozygous null mice at a Mendelian frequency. However, the breeding between homozygous females and males (PTX3 -/-) is completely infertile. Breeding results indicated that homozygous males are normally fertile when mated with wild type (PTX3 +/+) or heterozygous (PTX3 +/-) females, while PTX3 -/- females are always infertile, independently from the male genotype. Mating experiments indicated that there were no differences between PTX3 -/- and PTX3 +/+ females in the frequency of copulation plugs after spontaneous mating during a four days period or after superovulation (Table 1). The number of spontaneously ovulated eggs (Table 1) (average 7 per mouse, n=4, in PTX3 +/- and 7.8 per mouse, n=8, in PTX 3 -/- mice) or hormonally induced ovulated eggs (average 35 per mouse, n=9, in PTX3 +/- and 27 per mouse, n=18, in PTX3 -/- mice) was comparable in +/+ and -/- mice. Data are from one representative experiment of four performed. Oocyte and zona pellucida morphology were normal, and the first polar bodies were observed in about 50% of oocytes obtained 16 hr after human chorionic gonadotropin (hCG) treatment from both PTX3 +/+ and PTX3 -/- mice (Table 1). These data indicate that ovulation and oocyte maturation are normal and are not the cause of infertility. In contrast, morphological abnormalities of the cumuli oophori collected from the oviduct of PTX3 -/- mice (Figs. 1 B and 1 D) were consistently observed, since the granulosa cells were loosely associated to the oocytes and did not form the corona radiata. PTX3 -/- derived cumuli were unstable *in vitro* and granulosa cells spontaneously detached from the oocytes in a short time (15-60 min in PTX3 -/- versus several hours in PTX3 +/+ cumuli) after collection (14-16 hr post hCG, or at day 0.5 after natural mating), quickly leading to oocyte denudation.

**TABLE 1. Normal mating frequency and ovulation in PTX3 -/- mice**

| | | | PTX3 +/+ | PTX3 -/- | P value |
|---|---|---|---|---|---|
| Mating frequency Spontaneus (a) | | | | | |
| | 1 st day | | 4/9 | 2/10 | NS |
| | 2nd day | | 2/5 | 2/8 | NS |
| | 3th day | | 2/3 | 2/5 | NS |
| After superovulation | | | 4/4 | 8/8 | NS |
| Ovulation Spontaneus (b): mice ovulating | | | 4/4 | 5/5 | NS |
| | | eggs per mouse | 7 | 7.8 | - # |
| After superovulation: | | mice ovulating | 5/5 | 6/6 | NS |
| | | eggs per mouse | 37.8 | 33.3 | - |
| Presence of polar body in in ovulated eggs (c) | | | 53/98 | 54/109 | NS |
| | | | (54%) | (49%) | - |

| | | | | | |
|---|---|---|---|---|---|
| (a) Females were housed with males for a four days period and checked daily for the presence of plugs. | | | | | |
| (b) Ovulation was analyzed in females with plugs. | | | | | |
| (c) The presence of the first polar body was assessed in oocytes recovered 15 hr after HCG treatment. | | | | | |
| NS, not significantly different (p<0.05) from control PTX3 +/+ mice by Fischer's exact test. | | | | | |
| # Numbers refer to pooled samples from PTX3 +/+ or PTX3 -/- mice. A similar lack of difference was observed in four experiments with 5-7 mice. | | | | | |

To understand whether and when pregnancy was interrupted, zygotes and embryos were collected at different time points after mating after spontaneous or hormonally-induced ovulation. No oocytes developing to the two-cell stage in vivo (day 1.5) (Table 2) nor oocytes with two pronuclei (day 0.5) were ever observed, even if viable sperm were found in the oviduct of deficient mice. To further identify the cause(s) of infertility, PTX3 +/+ blastocysts were transferred to PTX3 -/pseudopregnant females, but normal pregnancy and delivery were observed. This excludes defects in implantation and subsequent processes.

**TABLE 2. Fertilization in PTX3 -/- mice**

| Fertilization | | PTX3 +/+ | PTX3 -/- | P value |
|---|---|---|---|---|
| In Vivo | | | | |
| Eggs fertilized over total (a) | | | | |
| | Spontaneus ovulation: | 17/28 (60%) | 0/39 (0%) | <0.0001# |
| | After superovulation: | 81/162 (50 %) | 0/192 (0%) | <0.0001 |

| In Vitro | | | | |
|---|---|---|---|---|
| After zona pellucida removal (b) | | 21/27 (77%) | 21/31 (68%) | NS⁺ |
| Using intact cumuli oophori (c) | | 79/189 (41.8%) | 68/169 (40%) | NS |

| | | | | |
|---|---|---|---|---|
| (a) Embryos were collected at 1.5 days postcoitum, at the two-cell stage. | | | | |
| (b) Fusion was assessed by the dye transfer technique 4 hr after insemination. | | | | |
| (c) Two cells embryos were counted the day after insemination. | | | | |
| # Fischer's exact test. | | | | |
| NS, not significantly different (p<0.05) from control PTX3 +/+ mice. | | | | |

To evaluate whether PTX3 -/- oocytes could be fertilized, in vitro fertilization (IVF) was performed using wild-type sperm from adult males to inseminate PTX3 +/+ or PTX3 -/- oocytes (Table 2). IVF was first conducted with oocytes freed from the zona pellucida and stained with the DNA-specific fluorochrome Hoechst 33258 to observe the fusion. Under these conditions, normal sperm binding to PTX3 -/oocyte plasma membrane and comparable fusing ability of PTX3 +/+ (77%) and PTX3 -/- (68%) oocytes with sperm (Table 2) were observed. These results suggested that sperm-egg binding and fusion can occur in the absence of PTX3. Intact cumuli collected 13-15 hr after hCG treatment were inseminated and fertilization of PTX3 -/- oocytes and progression to the two-cell stage were observed with a frequency comparable with PTX3 +/+ oocytes (Table 2). These data confirm that oocyte quality is normal in PTX3 deficient mice. Since the cumulus oophorus plays a critical role for in vivo, but not for in vitro fertilization, these results suggest that abnormalities in the cumulus underlie the infertility of PTX3 -/- females.

The expression of PTX3 mRNA in ovarian tissues has been investigated by Northern blotting and in situ hybridization. After hormonally-induced superovulation, PTX3 mRNA expression (assessed by Northern blotting in whole tissue) starts 2 hr after hCG treatment and lasts until 12-14 hr (see Fig. 2A), corresponding to preovulatory expansion until a few hours after ovulation [20]. Granulosa cells obtained by hyaluronidase treatment of cumuli oophori and separation from oocytes expressed PTX3 transcripts.

Expression under normal condition in the absence of superovulation was investigated by in situ hybridization. In situ hybridization of organs from untreated females (Fig. 2B) confirmed the expression of PTX3 mRNA in the ovary, confined to granulosa cells of mature follicles, with no evidence of transcription in oocytes.

PTX3 protein expression in ovarian tissues was then analyzed. Western blotting indicated that PTX3 was associated with PTX3 +/+ cumuli (in particular with extracellular matrix) because hyaluronidase treatment, which separates cumulus cells from oocytes, abolished immune reactivity (Fig. 2C). Immunofluorescence analysis of PTX3 +/+ and -/- cumuli oophori collected after hormonally-induced superovulation (13-15 hr after hCG) confirmed the association of PTX3 with cumulus intercellular matrix (Fig. 2D).

These data suggest that sterility caused by PTX3 deficiency is due to a lack of oocyte fertilization, as PTX3 deficiency does not affect other steps of reproduction, from mating to ovulation, implantation, and pregnancy. PTX3 transcripts are expressed in the normal ovary exclusively by the granulosa cells of mature follicles, as well as by separated granulosa cells, but not by oocytes. PTX3 mRNA expression is induced in total ovarian tissues following hormonally-induced superovulation. Finally, PTX3 protein has been identified in the extracellular matrix of isolated cumuli, presumably produced by granulosa cells. Analysis of PTX3 -/mice has identified an abnormal cumulus oophorus as a determinant of infertility. Cumuli oophori from PTX3 -/- females showed morphological abnormalities. They lacked a well-defined corona radiata and, upon in vitro culture, rapidly detached from oocytes. The "fragility" of PTX3 deficient cumuli may reflect a structural role of PTX3 in this peculiar matrix or an alteration in regulatory mechanisms of matrix dissolution. These results identify PTX3 as a novel constituent of the extracellular matrix of the cumulous oophorus, playing a key role in fertility. The cumulus oophorus, though not essential in vitro, plays a key role for in vivo fertilization. Therefore, the abnormalities of the cumulus oophorus are likely to be involved in the infertility of PTX3 -/- female mice.

### MATERIALS AND METHODS

### Generation of PTX3 -/- mice

A genomic DNA fragment of 8.5 kb encompassing exons 1 through 2 of the mouse PTX3 gene was used to integrate the IRES-LacZ cassette followed by the PGK-neomycin resistance gene from the pWH9 plasmid in exon 1 at a location 71 bp downstream of the first coding ATG. Methods for the culture, selection, and identification of ES cells were performed as described [20]. Five independently targeted R1 ES cell clones were identified by Southern blot hybridization, using probe A (EcoRI/EcoRV 750 bp fragment in the second intron). No evidence for random integration was detected with the probe B (from the neomycin resistance gene). Two ES cell clones were injected into C57BI/6 blastocysts. For genotyping of mice, DNA derived from tail biopsies was amplified by polymerase chain reaction with two primers sets (Primer Set 1: 5'-AGCAATGCACCTCCCTGCGAT-3', SEQ ID NO:7;5'-TCCTCGGTGGGATGAAGTCCA-3' SEQ ID NO:8; Primer Set 2: 5'-CTGCTCTTTACTGAAGGCTC-3', SEQ ID NO:9; 5'-TCCTCGGTGGGATGAAGT CCA-3, SEQ ID NO:10) that detected the wild type or targeted allele, respectively. Phenotypic analysis was performed on the two lines derived from independent clones, and results were confirmed in a 129Sv-C57BI/6 mixed and 129Sv inbred genetic background. PTX3 +/+ mice were 129Sv-C57BI/6 PTX3 -/- littermates, or 129Sv or C57BI/6 mice obtained from Charles River, Calco, Italy.

Procedures involving animals and their care in conformed with institutional guidelines in compliance with national (4D.L. N.116, G.U., suppl. 40, 18-2-1992) and international law and policies (EEC Council Directive 86/609, OJ L 358, 1, 12-12-1987; NIH Guide for the Care and Use of Laboratory Animals, U.S. National Research Council, 1996). All efforts were made to minimize the number of animals used and their suffering.

### PTX3 mRNA and protein

RNA was extracted from cells and purified using TRIZOL reagent (GIBCO BRL). Northern blotting, probe labeling, and hybridization (binding and washing) conditions were performed as described [21].

In situ hybridation: Cryostat sections (13 µm) recovered from wildtype and PTX3 -/- ovaries fixed with paraformaldehyde 4% and frozen in liquid nitrogen were used to perform the in situ hybridization as described [22]. Briefly, slides pemeabilized with proteinase K and 0.2N HCl, were incubated at 65°C overnight with a radioactively-labelled riboprobe made from PTX3 cDNA containing vector (pBluescript) using a Stratagene RNA transcription kit. Subsequently, specimens were washed with formamide-containing buffer, air dried, dipped in photographic emulsion and incubated at 4°C in a dark box for at least 10 days. After developing, the slides were counterstained with a solution of 2 µg/ml Hoechst 33258 dye. For Western blot analysis, total cell extracts obtained from intact cumuli oophori, cumulus cells, or oocytes collected from superovulated females were separated by SDS-polyacrylamide gel electrophoresis (Page), electroblotted onto nitrocellulose filters (Hybond ECL, Amersham), and labeled with a purified biotinylated anti-murine PTX3 polyclonal hamster serum (1 µg/ml) followed by strept-avidin-HRP (BIOSPA, Italy). Labeled proteins were detected by enhanced chemi-luminescence (ECL, Amersham).

### Oocyte and embryo collection, in vitro fertilization, and embryo transfer

Cumuli oophori, zygotes, and embryos were recovered from the oviduct or uterus of untreated females after natural mating [20]. Superovulation was induced by treatment with 5 units of pregnant mare serum (PMS, Folligon, Intervet) and with 5 units of human chorionic gonadotropin (hCG, Corulon, Intervet) 48 hr later. Cumuli oophori were collected at different time after mating or 13-15 hr after hCG treatment. Cumulus cells and oocytes were separated by hyaluronidase treatment [20].

In vitro fertilization (IVF) of eggs obtained from superovulated females was performed with intact cumuli oophori as described [20] or with zona pellucida free eggs [20] stained with 1 µg/ml Hoechst dye in M16 medium (Sigma) [23] and sperm from BDF males. Fertilization and sperm-egg fusion were assessed by counting two-cell stage embryos the day after insemination of intact cumuli oophori and by counting eggs with fluorescent fertilizing sperm 4 hr after insemination of zona pellucida-free eggs.

Embryo transfer was performed as described [20], using 3.5 day PTX3 +/+ blastocysts implanted in the uterus of 2.5 days pseudopregnant PTX3 -/- females.

### REFERENCES

1. Emsley et al., *Structure of pentameric human serum amyloid P component.* Nature, 1994. **367:**338-345.
2. Baumann & Gauldie, *The acute phase response.* Immunol. Today, 1994. **15**:74-80.
3. Steel & Whitehead, *The major acute phase reactants: C-reactive protein, serum amyloid P component and serum amyloid A protein.* Immunol. Today, 1994. **15**:81-88.
4. Breviario et al., *Inter*/*eukin-1-inducible genes in endothelial cells. Cloning of a new gene related to C-reactive protein and serum amyloid P component. J.* Biol. Chem., 1992. **267**:22190-22197.
5. Lee et al., *TSG-14, a tumor necrosis factor- and IL-1-inducible protein, is a novel member of the pentaxin family of acute phase proteins.* J. Immunol., 1993. **150**:1804-1812.
6. Lee et al., *Relationship of TSG-14 protein to the pentraxin family of major acute phase proteins.* J. lmmunol., 1994. **153**:3700-3707.
7. Vidal Alles et al., *Inducible expression of PTX3, a new member of the pentraxin family, in human mononuclear phagocytes.* Blood, 1994. **84**:3483-3493.
8. Introna et al., *Cloning of mouse PTX3, a new member of the pentraxin gene family expressed at extrahepatic sites.* Blood, 1996. **87**:1862-1872.
9. Bottazzi et al., *Multimer formation and ligand recognition by the long pentraxin PTX3 - Similarities and differences with the short pentraxins C-reactive protein and serum amyloid P component.* J. Biol. Chem., 1997. **272**:32817-32823.
10. Muller et al., *Circulating levels of the long pentraxin PTX3 correlate with severity of infection in critically ill patients.* Crit. Care Med. 2001. **29**:1404-1407.
11. Noland et al., *The sperm acrosomal matrix contains a novel member of the pentaxin family of calcium-dependent binding proteins.* J. Biol. Chem., 1994. **269**:32607-32614.
12. Reid & Blobel, *Apexin, an acrosomal pentaxin.* J. Biol. Chem., 1994. **269**:32615-32620.
13. Seery et al., *Identification of a novel member of the pentraxin family in Xenopus laevis.* Proc. R. Soc. Lond. B. Biol. Sci., 1993. **253**:263-270.
14. Schlimgen et al., *Neuronal pentraxin, a secreted protein with homology to acute phase proteins of the immune system.* Neuron, 1995. **14**:519-526.
15. Omeis et al., *Mouse and human neuronal pentraxin 1 (NPTX1): Conservation, genomic structure, and chromosomal localization.* Genomics, 1996. **36**:543-545.
16. Hsu & Perin, *Human neuronal pentraxin II (NPTX2): Conservation, genomic structure, and chromosomal localization.* Genomics, 1995. **28**:220-227.
17. Tsui et al., *Narp, a novel member of the pentraxin family, promotes neurite outgrowth and is dinamically regulated by neuronal activity.* J. Neurosci., 1996. **15**:2463-2478.
18. Dodds et al., *Neuronal pentraxin receptor, a novel putative integral membrane pentraxin that interacts with neuronal pentraxin 1 and 2 and taipoxin-associated calcium-binding protein 49.* J. Biol. Chem., 1997. **272**:21488-21494.
19. Kirkpatrick et al., *Biochemical interactions of the neuronal pentraxins. Neuronal pentraxin (NP) receptor binds to taipoxin and taipoxin-associated calcium-binding protein **49** via NP1 and NP2.* J. Biol. Chem., 2000. **275**:17786-17792.
20. Hogan et al., *Manipulating the Mouse Embryo. A laboratory manual.* 2nd Ed., 1994: Cold Spring Harbor Laboratory Press.
21. Introna et al., *Treatment of murine peritoneal macrophages with bacterial lipopolysaccharide alters expression of c-fos and c-myc oncogenes.* J. lmmunol., 1986. **137**:2711-2715.
22. Biffo & Tolosano, *The use of radioactively labelled riboprobes for in situ hybridization: Background and examples of application.* Liver, 1992. **12**:230-237.
23. Conover & Gwatkin, *Pre-loading of mouse oocytes with DNA-specific fluorochrome (Hoechst 33342) permits rapid detection of sperm-oocyte fusion.* J. Reprod. Fertil.,1988. **82**:681-690.

All modifications and substitutions that come within the meaning of the claims and the range of their legal equivalents are to be embraced within their scope. A claim using the transition "composing" allows the inclusion of other elements to be within the scope of the claim; the invention is also described by such claims using the transitional phrase "consisting essentially of (i.e., allowing the inclusion of other elements to be within the scope of the claim if they do not materially affect operation of the invention) and the transition "consisting" (i.e., allowing only the elements listed in the claim other than impurities or inconsequential activities which are ordinarily associated with the invention) instead of the "comprising" term. Any of the three transitions-can be used to claim the invention

It should be understood that an element described in this specification should not be construed as a limitation of the claimed invention unless it is explicitly recited in the claims. Thus, the claims are the basis for determining the scope of legal protection granted instead of a limitation from the specification which is read into the claims.

In contradistinction, the prior art is explicitly excluded from the invention to the extent of specific embodiments that would anticipate the claimed invention or destroy novelty. In certain embodiments, the genus of polynucleotides or polypeptides may be recited in the claims with the proviso that native nucleic acids or proteins are excluded (e.g., having a nucleotide or amino acid sequence which is not given in the sequence listing). For example, the degeneracy of the genetic code may be used to provide a polynucleotide having a nucleotide sequence encoding SEQ ID NO:2. but which is not SEQ ID NO:1. Similarly, a PTX3 polypeptide may be provided that is fernctionatly equivalent but not identical to the mouse and/or human protein (e.g., at least 90% identical) by changing one or more of the amino acid residues of SEQ ID NO:2.

It would be apparent to a person of skill In this art that the invention can be embodied in other specific forms without departing from its spirit or essential characteristics. The described embodiments should be considered only as illustrative, not restrictive, because the scope of the legal protection provided for the invention will be indicated by the appended claims rather than by this specifcation.

## Claims

1. Use of the recombinant human PTX3 for preparing a medicament for increasing the reproductive ability in a female subject with a defect in reproduction.

2. Use according to claim 1, wherein said subject is a female patient or an animal.

3. Use according to claims 1-2 in which the medicament is to be administered systemically.

4. Use according to claim 1-2 in which the medicament is to be administered locally.

5. A method for the determination the reproductive ability in human female comprising the detection of the presence of PTX3 protein as diagnostic marker in fluids or tissues withdrawn from said human.

## Patentansprüche

1. verwendung des rekombinanten menschlichen PTX3 zur Herstellung eines Medikaments zur Erhöhung der Reproduktionsfähigkeit eines weibliches Subjekts mit einem Defekt in der Reproduktion.

2. Verwendung gemäß Anspruch 1, wobei das Subjekt ein weiblicher Patient oder ein Tier ist.

3. Verwendung gemäß den Ansprüchen 1 bis 2, worin das Medikament systemisch zu verabreichen ist.

4. Verwendung gemäß den Ansprüchen 1 bis 2, worin das Medikament lokal zu verabreichen ist.

5. Verfahren zur Bestimmung der Reproduktionsfähigkeit einer menschlichen Frau, umfassend den Nachweis der Gegenwart des PTX3-Protein als diagnostischen Marker in Fluids oder Geweben, die der Frau entnommen wurden.

## Revendications

1. Utilisation de PTX3 recombinante humaine pour la préparation d'un médicament destiné à augmenter la capacité reproductrice chez un sujet femelle présentant une anomalie de la reproduction.

2. Utilisation selon la revendication 1, dans laquelle ledit sujet est une patiente ou un animal.

3. Utilisation selon les revendications 1 ou 2, dans laquelle le médicament est destiné à être administré par voie systémique.

4. Utilisation selon les revendications 1 ou 2, dans laquelle le médicament est destiné à être administré par voie locale.

5. Méthode de détermination de la capacité reproductrice chez une femme comprenant la détection de la présence de la protéine PTX3 en tant que marqueur de diagnostic dans des fluides ou des tissus prélevés de ladite femme.


### SEQUENCE LISTING

<110> Sigma-Tau Industrie Farmaceutiche Riunite S.P.A.
<120> Long Pentaxin PTX3 and Female Sterility
<130> O12-ST-01-US
<140>
   <141>
<150> US 60/309,472
   <151> 2001-08-03
<160> 11
<170> PatentIn version 3.1
<210> 1
   <211> 1837
   <212> DNA
   <213> Homo sapiens
<300>
   <301> Breviario et al.
   <302> Interleukin-1 Inducible Genes in Endothelial Cells
   <303> Journal of Biological Chemistry
   <304> 267
   <305> 31
   <306> 22190-22197
   <307> 1992-11-05
   <308> X636613
   <309> 1993-07-29
<400> 1
   ctcaaactca gctcacttga gagtctcctc ccgccagctg tggaaagaac tttgcgtctc      60
   tccagcaatg catctccttg cgattctgtt ttgtgctctc tggtctgcag tgttggccga     120
   gaactcggat gattatgatc tcatgtatgt gaatttggac aacgaaatag acaatggact     180
   ccatcccact gaggacccca cgccgtgcga ctgcggtcag gagcactcgg aatgggacaa     240
   gctcttcatc atgctggaga actcgcagat gagagagcgc atgctgctgc aagccacgga     300
   cgacgtcctg cggggcgagc tgcagaggct gcgggaggag ctgggccggc tcgcggaaag     360
   cctggcgagg ccgtgcgcgc cgggggctcc cgcagaggcc aggctgacca gtgctctgga     420
   cgagctgctg caggcgaccc gcgacgcggg ccgcaggctg gcgcgtatgg agggcgcgga     480
   ggcgcagcgc ccagaggagg cggggcgcgc cctggccgcg gtgctagagg agctgcggca     540
   gacgcgagcc gacctgcacg cggtgcaggg ctgggctgcc cggagctggc tgccggcagg     600
   ttgtgaaaca gctattttat tcccaatgcg ttccaagaag atttttggaa gcgtgcatcc     660
   agtgagacca atgaggcttg agtcttttag tgcctgcatt tgggtcaaag ccacagatgt     720
   attaaacaaa accatcctgt tttcctatgg cacaaagagg aatccatatg aaatccagct     780
   gtatctcagc taccaatcca tagtgtttgt ggtgggtgga gaggagaaca aactggttgc     840
   tgaagccatg gtttccctgg gaaggtggac ccacctgtgc ggcacctgga attcagagga     900
   agggctcaca tccttgtggg taaatggtga actggcggct accactgttg agatggccac     960
   aggtcacatt gttcctgagg gaggaatcct gcagattggc caagaaaaga atggctgctg    1020
   tgtgggtggt ggctttgatg aaacattagc cttctctggg agactcacag gcttcaatat    1080
   ctgggatagt gttcttagca atgaagagat aagagagacc ggaggagcag agtcttgtca    1140
   catccggggg aatattgttg ggtggggagt cacagagatc cagccacatg gaggagctca    1200
   gtatgtttca taaatgttgt gaaactccac ttgaagccaa agaaagaaac tcacacttaa    1260
   aacacatgcc agttgggaag gtctgaaaac tcagtgcata ataggaacac ttgagactaa    1320
   tgaaagagag agttgagacc aatctttatt tgtactggcc aaatactgaa taaacagttg    1380
   aaggaaagac attggaaaaa gcttttgagg ataatgttac tagactttat gccatggtgc    1440
   tttcagttta atgctgtgtc tctgtcagat aaactctcaa ataattaaaa aggactgtat    1500
   tgttgaacag agggacaatt gttttacttt tctttggtta attttgtttt ggccagagat    1560
   gaattttaca ttggaagaat aacaaaataa gatttgttgt ccattgttca ttgttattgg    1620
   tatgtacctt attacaaaaa aaatgatgaa aacatattta tactacaagg tgacttaaca    1680
   actataaatg tagtttatgt gttataatcg aatgtcacgt ttttgagaag atagtcatat    1740
   aagttatatt gcaaaaggga tttgtattaa tttaagacta tttttgtaaa gctctactgt    1800
   aaataaaata ttttataaaa ctaaaaaaaa aaaaaaa                             1837
<210> 2
   <211> 381
   <212> PRT
   <213> Homo sapiens
<220>
   <221> SIGNAL PEPTIDE
   <222> (1)..(17)
   <223>
<220>
   <221> MAT_PEPTIDE
   <222> (18)..(381)
<300>
   <301> Breviario et al.
   <302> Interleukin-1 Inducible Genes in Endothelial Cells
   <303> Journal of Biological Chemistry
   <304> 267
   <305> 31
   <306> 22190-22197
   <307> 1992-11-05
   <308> CAA45158
   <309> 1993-07-29
<400> 2
   Met His Leu Leu Ala Ile Leu Phe Cys Ala Leu Trp Ser Ala Val Leu
           -15                 -10                 -5
   Ala Glu Asn Ser Asp Asp Tyr Asp Leu Met Tyr Val Asn Leu Asp Asn
   -1  1               5                   10                  15
   Glu Ile Asp Asn Gly Leu His Pro Thr Glu Asp Pro Thr Pro Cys Asp
                   20                  25                  30
   Cys Gly Gln Glu His Ser Glu Trp Asp Lys Leu Phe Ile Met Leu Glu
               35                  40                  45
   Asn Ser Gln Met Arg Glu Arg Met Leu Leu Gln Ala Thr Asp Asp Val
           50                  55                  60
   Leu Arg Gly Glu Leu Gln Arg Leu Arg Glu Glu Leu Gly Arg Leu Ala
       65                  70                  75
   Glu Ser Leu Ala Arg Pro Cys Ala Pro Gly Ala Pro Ala Glu Ala Arg
   80                  85                  90                  95
   Leu Thr Ser Ala Leu Asp Glu Leu Leu Gln Ala Thr Arg Asp Ala Gly
                   100                 105                 110
   Arg Arg Leu Ala Arg Met Glu Gly Ala Glu Ala Gln Arg Pro Glu Glu
               115                 120                 125
   Ala Gly Arg Ala Leu Ala Ala Val Leu Glu Glu Leu Arg Gln Thr Arg
           130                 135                 140
   Ala Asp Leu His Ala Val Gln Gly Trp Ala Ala Arg Ser Trp Leu Pro
       145                 150                 155
   Ala Gly Cys Glu Thr Ala Ile Leu Phe Pro Met Arg Ser Lys Lys Ile
   160                 165                 170                     175
   Phe Gly Ser Val His Pro Val Arg Pro Met Arg Leu Glu Ser Phe Ser
                   180                 185                 190
   Ala Cys Ile Trp Val Lys Ala Thr Asp Val Leu Asn Lys Thr Ile Leu
               195                 200                 205
   Phe Ser Tyr Gly Thr Lys Arg Asn Pro Tyr Glu Ile Gln Leu Tyr Leu
           210                 215                 220
   Ser Tyr Gln Ser Ile Val Phe Val Val Gly Gly Glu Glu Asn Lys Leu
       225                 230                 235
   Val Ala Glu Ala Met Val Ser Leu Gly Arg Trp Thr His Leu Cys Gly
   240                 245                 250                     255
   Thr Trp Asn Ser Glu Glu Gly Leu Thr Ser Leu Trp Val Asn Gly Glu
                   260                 265                 270
   Leu Ala Ala Thr Thr Val Glu Met Ala Thr Gly His Ile Val Pro Glu
               275                 280                 285
   Gly Gly Ile Leu Gln Ile Gly Gln Glu Lys Asn Gly Cys Cys Val Gly
           290                 295                 300
   Gly Gly Phe Asp Glu Thr Leu Ala Phe Ser Gly Arg Leu Thr Gly Phe
       305                 310                 315
   Asn Ile Trp Asp Ser Val Leu Ser Asn Glu Glu Ile Arg Glu Thr Gly
   320                 325                 330                     335
   Gly Ala Glu Ser Cys His Ile Arg Gly Asn Ile Val Gly Trp Gly Val
                   340                 345                 350
   Thr Glu Ile Gln Pro His Gly Gly Ala Gln Tyr Val Ser
               355                 360
<210> 3
   <211> 1841
   <212> DNA
   <213> Mus musculus
<300>
   <301> Introna et al.
   <302> Cloning of Mouse PTX3
   <303> Blood
   <304> 87
   <305> 5
   <306> 1862-1872
   <307> 1996-03-01
   <308> X83601
<309> 1996-01-10
<400> 3
   actcctgcct cacactatct ctcccgggct caaactcgga tcactgtaga gtctcgcttc      60
   ttcccctgcg gctgcgaacg aaatttcgcc tctccagcaa tgcacctccc tgcgatcctg     120
   ctttgtgctc tctggtctgc agtagtggct gagacctcgg atgactacga gctcatgtat     180
   gtgaatttgg acaacgaaat agacaatgga cttcatccca ccgaggaccc cacgccatgc     240
   gactgccgcc aggagcactc ggagtgggac aagctgttca tcatgctgga gaactcgcag     300
   atgcgggagg gcatgctgtt gcaggccacc gacgacgtcc tccgtggaga gctgcagcgg     360
   ctgcgggcag agctggggcg gctggcgggc ggcatggcga ggccgtgcgc agccggtggc     420
   cccgcagacg ccaggctggt gcgggcgctg gagccgctgc tgcaggagag ccgtgacgcg     480
   agcctcaggc tggcgcgcct ggaggacgcg gaggcgcggc gacccgaggc gacagtgcct     540
   ggcctaggcg ctgtgctgga ggaactgcgg cggacgcgcg ccgacctgag cgccgtgcag     600
   agctgggtcg cccgccactg gctgcccgca ggttgtgaaa cagcaatttt cttcccaatg     660
   cgttcgaaga agatttttgg aagcgtgcat cctgtgagac caatgaagct tgaatctttt     720
   agtacttgca tttgggtcaa agccacagat gtattaaaca aaaccatcct gttttcttat     780
   ggcacaaagt ggaaccccta tgagattcag ctgtacctca gttcccagtc cctagtgttg     840
   gtggtgggtg gaaaggagaa caagctggct gcagacactg tggtgtccct ggggaggtgg     900
   tcccacctgt gtggcacctg gagttcagag caggggagca tgtccctgtg ggcaaacggg     960
   gagctggtgg ctaccactgt agagatggcc aaaagtcact ctgttcctga gggtggactc    1020
   ctacagattg gccaagaaaa gaatggttgc tgtgtaggtg gggctttgga cgaatcatta    1080
   gcattttctg gaagaatcac aggcttcaat atctgggatc gggttctcag cgaggaggag    1140
   atacgggcca gtggaggagt cgaatcctgt cacatccggg gaaatgtcgt cgggtgggga     1200
   gtcacagaga ttcaggcgca cggaggagcc cagtatgttt cttaagtgtt gtgaaaatct    1260
   acttgaagcc aaaggagact cacattttaa atatgccagt tggaaaagtc tgaaaacttc    1320
   ggtgcgtaat agacgaatga aggagagact tgagattgtc tttgtttatc ttggcaaaat    1380
   actgaataca cagttgaagg gaaggcttga gagagggctc cgggatgttg ttactaagcc    1440
   ttatactgtg gtgctttcag attaatgtct gcctctgtca gataaaccct cagataacta    1500
   aacatgactg gactctgaac agagggacga ttgtgtgact tttttttttt tttattttgg    1560
   ttaattttat tttggccaga gacattttta tattggaaga ataacaaaac aagctctgtt    1620
   gcccattgtt cattctttct ggtgtgtatt ttgtgacaaa agagatgatg agaaaaccat    1680
   aattatacca caaagtgact tattaacgaa cataaatgta gcttacgtgt tataatccaa    1740
   tccatttggg agaaggtagt tgtgtaattt atattgtgaa atgtaattgt attaatttta    1800
   tttttgtaaa agtctactgt aaataaattg ttttataaag c                        1841
<210> 4
   <211> 381
   <212> PRT
   <213> Mus musculus
<220>
   <221> SIGNAL PEPTIDE
   <222> (1)..(17)
   <223>
<220>
   <221> MAT PEPTIDE
   <222> (18) .. (381)
   <223>
<300>
   <301> Introna et al.
   <302> Cloning of Mouse PTX3
   <303> Blood
   <304> 87
   <305> 5
   <306> 1862-1872
   <307> 1996-03-01
   <308> CAA58580
<309> 1996-01-10
<400> 4
   Met His Leu Pro Ala Ile Leu Leu Cys Ala Leu Trp Ser Ala Val Val
   -15 -10 -5
   Ala Glu Thr Ser Asp Asp Tyr Glu Leu Met Tyr Val Asn Leu Asp Asn
   -1 1 5 10 15
   Glu Ile Asp Asn Gly Leu His Pro Thr Glu Asp Pro Thr Pro Cys Asp
   20 25 30
   Cys Arg Gln Glu His Ser Glu Trp Asp Lys Leu Phe Ile Met Leu Glu
   35 40 45
   Asn Ser Gln Met Arg Glu Gly Met Leu Leu Gln Ala Thr Asp Asp Val
   50 55 60
   Leu Arg Gly Glu Leu Gln Arg Leu Arg Ala Glu Leu Gly Arg Leu Ala
   65 70 75
   Gly Gly Met Ala Arg Pro Cys Ala Ala Gly Gly Pro Ala Asp Ala Arg
   80 85 90 95
   Leu Val Arg Ala Leu Glu Pro Leu Leu Gln Glu Ser Arg Asp Ala Ser
   100 105 110
   Leu Arg Leu Ala Arg Leu Glu Asp Ala Glu Ala Arg Arg Pro Glu Ala
   115 120 125
   Thr Val Pro Gly Leu Gly Ala Val Leu Glu Glu Leu Arg Arg Thr Arg
   130 135 140
   Ala Asp Leu Ser Ala Val Gln Ser Trp Val Ala Arg His Trp Leu Pro
   145 150 155
   Ala Gly Cys Glu Thr Ala Ile Phe Phe Pro Met Arg Ser Lys Lys Ile
   160 165 170 175
   Phe Gly Ser Val His Pro Val Arg Pro Met Lys Leu Glu Ser Phe Ser
   180 185 190
   Thr Cys Ile Trp Val Lys Ala Thr Asp Val Leu Asn Lys Thr Ile Leu
   195 200 205
   Phe Ser Tyr Gly Thr Lys Trp Asn Pro Tyr Glu Ile Gln Leu Tyr Leu
   210 215 220
   Ser Ser Gln Ser Leu Val Leu Val Val Gly Gly Lys Glu Asn Lys Leu
   225 230 235
   Ala Ala Asp Thr Val Val Ser Leu Gly Arg Trp Ser His Leu Cys Gly
   240 245 250 255
   Thr Trp Ser Ser Glu Gln Gly Ser Met Ser Leu Trp Ala Asn Gly Glu
   260 265 270
   Leu Val Ala Thr Thr Val Glu Met Ala Lys Ser His Ser Val Pro Glu
   275 280 285
   Gly Gly Leu Leu Gln Ile Gly Gln Glu Lys Asn Gly Cys Cys Val Gly
   290 295 300
   Gly Gly Phe Asp Glu Ser Leu Ala Phe Ser Gly Arg Ile Thr Gly Phe
   305 310 315
   Asn Ile Trp Asp Arg Val Leu Ser Glu Glu Glu Ile Arg Ala Ser Gly
   320 325 330 335
   Gly Val Glu Ser Cys His Ile Arg Gly Asn Val Val Gly Trp Gly Val
   340 345 350
   Thr Glu Ile Gln Ala His Gly Gly Ala Gln Tyr Val Ser
   355 360
<210> 5
   <211> 1531
   <212> DNA
   <213> Homo sapiens
<220>
   <221> PROMOTER
   <222> (1)..(1317)
   <223>
<220>
   <221> PROTEIN_BIND
   <222> (1222)..(1231)
   <223> NF-kB
<300>
   <301> Basile et al.
   <302> Characterization of the Promoter for the Human Long Pentaxin PTX3
   <303> Journal of Biological Chemistry
   <304> 272
   <305> 13
   <306> 8172-8178
   <307> 1997-03-28
   <308> X97748
   <309> 1997-11-15
<400> 5
   gaattccccg gatctccctt ctaactctcc acctttggcc taagctttgc ttccacatgg     60
   tcatcaacat ttggtggtta tagaactaat aacccctatc tcacttcact cctatgccag    120
   aggggcccta gcatcagctc atgggattgt tgtttttgct ttcctctcta tctttggctc    180
   cgggattttc cccttacttt aatgggagct catctgtacc ttttaagttt ttattaatat    240
   catgtgaaca cagacctgta tatattgtta gaagcagaaa tctctaagtt tacttttaaa    300
   acatgatcct tgcctcgaaa ccttgtagaa taatataatg tccacataat accaagttat    360
   gaaaagaaac atacctaaat aactaaataa gtatattcct tttttccccc agcttttttt    420
   ccccattcta ggttacccag ttgtactgtg ttgtttgtca taggccgggt gaggtggctc    480
   acgtctgtaa tcctagcaat ttgggaggcg aaggcgggtg gatcgcctga ggtcaggagt    540
   tcgagaccag cctggctaac atggtgaaac cctgtctcta ctaaaaatac aaaaattaac    600
   tgggtgtggt ggcgggtgcc tgtaattcca gctacttggg aagctgaggt aggagaatcg    660
   cttgaaccca ggatgcggag gttgcagtga gccgagatca caccattgca ctccagcctg    720
   ggcaacaaga gcgaaattca gtctcaaaaa aaaaaattat ctataaaagt ataggtgcaa    780
   ctcctcaagt attaaagaca agatagctcg gattggactt gactttcaga gccataacta    840
   ttcttaatat gttggtttat cttggaatca gaccattttc agtttcaacc tgtaaaacag    900
   tgtacaaagg aaacatggaa agttttctat atataaaggg ttgtgaaata ataacagctc    960
   acagaaaatg ctgaaatgat gatttgcttc agtaccctct gaaatttctc ccctaccacc   1020
   cctccttcat ccccattgct atcaattcaa attacaacag ctaattctca ggagaacagt   1080
   agaagcccag tttctctcct ctttcccctc tgaccctcct ccaattaatc tgactgcagc   1140
   gtaaaccttt gcggtttaat attgtgcaac ttccacattt ccctcgctct cccacccagc   1200
   cccctccccc accaaattca ggggaactcc cgttaccgca gtgccaccag cattactcat   1260
   tcatccccat tcaggctttc ctcagcattt attaaggact ctctgctcca gcctctcact   1320
   ctcactctcc tccgctcaaa ctcagctcac ttgagagtct cctcccgcca gctgtggaaa   1380
   gaactttgcg tctctccagc aatgcatctc cttgcgattc tgttttgtgc tctctggtct   1440
   gcagtgttgg ccgagaactc ggatcattat catctcatgt atgtgaattt ggacaacgaa   1500
   atagacaatg gactccatcc cactgaggac c                                   1531
<210> 6
   <211> 2708
   <212> DNA
   <213> Mus musculus
<220>
   <221> PROMOTER
   <222> (1) .. (1373)
   <223>
<300>
   <301> Altmeyer et al.
   <302> Promoter Structure and Transcriptional Activation
   <303> Journal of Biological Chemistry
   <304> 270
   <305> 43
   <306> 25584-15590
   <307> 1995-10-27
   <308> U33842
   <309> 1995-10-27
<400> 6
   atcccagagg ctctctgtac tggcattagg acctcacagc accacatcag gtttcttaat 60
   gtggactcta gaaactgaac tcgagcccac agccttagga gaaaagcacc ttacaaagct 120
   gtggctccac actgcccttt aaacaatatc gtattgtctc atattgccat cgctttctga 180
   tggctttaac ggtttcaaac ataccctgtc tttagccgtg atctcaaata agtgaagctc 240
   ttgagcaggg gcctgatgcc ttttgacttt gtgttgattc atgcttatga tgccctgttc 300
   cctccgtgtc tagctatgtt taactgtgga ttcaattttt attggtgggt ggattggtac 360
   atgcatgtgc attccagatg cgtgagggca ctcaggccag gaaagccact catgagtctc 420
   tgtcaggagc agaggaattt acctatggaa atccaagagc agccttctga gaggcctggc 480
   ctgagggtag tacccctccc atcatgatca ggatgtgact ggtaaccctc cccctccatc 540
   tcctttgtat attggagact tgtatcagct caggggtatc ctctgggagt ggttccctct 600
   agatctgtgt agttttttag atcttgcttt atttggagtt tattctcatg ttttaatttt 660
   ttatcactat tattatgact tatcaacacc tatctaggta cttttcactg ggggaggggg 720
   caggttttac acacacacac acacacacac acacacacac acacacacac acagtcacta 780
   atgtaaaatt taaaacaggg accttgatag gatatgtcca agaataccca agcaccctaa 840
   agccactata ttcccgccct cactttcctg ttttactggg ttttgaccca gccatactgt 900
   gttttttagt tgctccacca gaggagtcaa gactagttag tcaagattga cttctagagt 960
   cataaaaatt cttaatgggt tattttggag tcacggaatc attttctata gcttggtctt 1020
   gagaaagtat ccaaaggaaa agtgaaaaaa aaaagttttc cataacttca ggggttgtgg 1080
   agtaatgaaa gctcacacca aatgccaaaa tgataattcg ccctgtacct ctgtgctcct 1140
   caccccccaa agcgctagca cttcaggtta cagcaactaa tcctcagggg caccagaaaa 1200
   gtccagcttc cctccccttc tccccctgac tcgcctctaa ttaatctgcc tgcagtgtgg 1260
   acctcggtgg tttaacattg tgcaacctct tcagctccct tgccctccca cccaaccccc 1320
   tcccccaaat ccaggggaac tccctcgcgc tgtgccaccg acattagtca ttcatccgct 1380
   catgctttgg agcgtttatt aagggcttca ctcctgcctc acactatctc tcccgggctc 1440
   aaactcggat cactgtagag tctcgcttct tcccctgcgg gtgcgaagca aatttcggct 1500
   ctccagcaat gcacctccct gcgatcctgc tttgtgctct ctggtctgca gtagtggctg 1560
   agacctcgga tgactacgag ctcatgtatg tgaatttgga caacgaaata gacaatggac 1620
   ttcatcccac cgaggaccgt aagttcattt ttaactctct cagcgtatca aaactacata 1680
   actcacttct gggggggcgc gattaacata attaacatag atagccaatg aagcaagcta 1740
   aaattatact ttatttgtga aagcaaggac tgggggaaaa aaggaaagca aggaaatatc 1800
   tgagaaaagc cagaggtttt aaattatttt tgtaacattt atgatgagtt aagttatacg 1860
   aaatctttaa ctgtttttag ctatattaat ggcattttct cagttagttt aacatgtcta 1920
   taaagaatag tctgtgtcat ctttgagttt acacgcacgc tgttttcaga gctatcctta 1980
   gaaggagagc gttgctgggg acaggctgaa acttggagtc accaagagtg caacccatgg 2040
   ccacccagga caagctgata acacttgtgt gtgtcctgcg ttctagccac gccatgcgac 2100
   tgcgcccagg agcactcgga gtgggacaag ctgttcatca tgctggagaa ctcgcagatg 2160
   cgggagggca tgctgttgca ggccaccgac gacgtcctcc gtggagagct gcagcggctg 2220
   cggtcagagc tgggccggct ggcgggcggc atggcgaggc cgtgcgcagc cggtggcccc 2280
   gcagacgcca ggctggtgcg ggcgctggag ccgctgctgc aggagagccg tgacgcgagc 2340
   ctcaggctgg cgcgcctgga ggacgcggag gcgcggcgac ccgaggcgac agtgcctggc 2400
   ctaggcgctg tgctggagga actgcggcgg acgcgctccg acctgagcgc cgtgcagagc 2460
   tgggtcgccc accactggct gcccgcaggt aagcccacgg tcggctctgt ccctagaggc 2520
   aagcttttgt gggaccctca cactcagagc cccagtactt ttcataggca cactcacaga 2580
   gctcacacca cgccaggcag ctcattgcct tttaaaagta tttccaagcc cgaggaaccc 2640
   aaaagaaaaa aacgaggatt taaaccatca gtctggaagt tgacgtcaga ggttcctgat 2700
   accggatc 2708
<210> 7
   <211> 21
   <212> DNA
   <213> Artificial Sequence
<220>
   <223> Oligonucleotide Primer
<400> 7
   agcaatgcac ctccctgcga t 21
<210> 8
   <211> 21
   <212> DNA
   <213> Artificial Sequence
<220>
   <223> Oligonucleotide Primer
<400> 8
   tcctcggtgg gatgaagtcc a 21
<210> 9
   <211> 20
   <212> DNA
   <213> Artificial Sequence
<220>
   <223> Oligonucleotide Primer
<400> 9
   ctgctcttta ctgaaggctc 20
<210> 10
   <211> 21
   <212> DNA
   <213> Artificial Sequence
<220>
   <223> Oligonucleotide Primer
<400> 10
   tcctcggtgg gatgaagtcc a 21
<210> 11
   <211> 8
   <212> PRT
   <213> Artificial Sequence
<220>
   <223> Consensus "pentraxin-like" sequence
<220>
   <221> MISC_FEATURE
   <222> (2)..(2)
   <223> any amino acid
<220>
   <221> MISC_FEATURE
   <222> (4)..(4)
   <223> any amino acid
<220>
   <221> MISC_FEATURE
   <222> (5)..(5)
   <223> Ser or Thr
<220>
   <221> MISC_FEATURE
   <222> (7)..(7)
   <223> any amino acid
<400> 11
   His Xaa Cys Xaa Xaa Trp Xaa Ser
   1               5